# EUROPEAN PATENT SPECIFICATION

(11) **EP 1 570 842 B1**
(45) Date of publication and mention of the grant of the patent: **05.09.2007**
(21) Application number: 04107001.2
(22) Date of filing: 27.12.2004
(51) Int. Cl.: A61K 9/20, A61K 31/737

(54) **Composition containing chondroitin sulphate**
Chondroitinsulfat enthaltende Zusammensetzung
Composition de chondroitin sulphate

(30) Priority: 30.12.2003 IT MI20032614
(43) Date of publication of application: 07.09.2005
(73) Proprietor: ALTERGON S.A., CH-6903 Lugano (CH)
(72) Inventor: Nocelli Luca, 21016 Luino (IT); Zoppetti, Giorgio, 20155 Milan (IT)
(74) Representative: Gervasi, Gemma

(56) References cited:
- EP-A- 0 704 216
- EP-A- 1 356 811
- WO-A-20/04012665
- US-A1- 2003 124 200
- US-A1- 2003 138 543

## Description

### FIELD OF THE INVENTION

The present invention concerns new pharmaceutical formulations containing a therapeutic quantity of a chondroitin sulfate salt, said formulation enabling a twofold therapeutic action to be obtained.

### PRIOR ART

Chondroitin sulfate belongs to the glycosaminoglycan family, these being high molecular weight polymer molecules whose units consist of disaccharides which, in living organisms, are bound to proteins by polar or co-valent bonds, to give structures of considerable biological importance.

Among the most important glycosaminoglycans noted are hyaluronic acid, keratan sulfate, heparin, dermatan sulfate and chondroitin sulfate.

This latter is present in various animal tissues and organs mixed with other glycosaminoglycans and, from a viewpoint of chemical structure, consists of repeating disaccharide units composed of a variously sulfated -D-N-acetyl-galactosamine residue bound to a, possibly sulfated, uronic acid (-D-glucuronic) residue. This endogenous molecule is commonly derived, by means of various extraction and purification procedures, from animal tissues containing it, in particular pig and cow tracheas, and from the cartilaginous skeletons of selacii.

Chondroitin sulfate is present in living organisms principally in the form of 2 isomers which differ only by the relocation of the sulfate ester bond positioned on the galactopyranosylamine residue: chondroitin sulfate A, or chondroitin-4-sulfate, consisting of disaccharide units [(1->4)-O-(β-D-glucopyranosyluronic acid)-(1->3)-O-2-N-acetamide-2-deoxy-β-D-galactopyranosyl-4-sulfate] and chondroitin sulfate C, or chondroitin 6-sulfate, consisting of disaccharide units [(1->4)-O-(β-D-glucopyranosyluronic acid)-(1->3)-O-2-N-acetamide-2-deoxy-β-D-galactopyranosyl-6-sulfate] (Murata K. and Yokoyama Y., Anal. Biochem. 149, 261, 1985). Non-sulfated, bi- and tri-sulfated disaccharides have also been identified, whose isolation depends mainly on the source of extraction and type of purification, and, in particular, on the type of animal, whether from tissue or organ, and the type of tissue itself etc.

In the description to follow, chondroitin sulfates, either 4-sulfate or 6-sulfate or their mixtures in combination with other existing forms, will be referred to generically as chondroitin sulfate.

Evidence for the therapeutic activity of this active principle dates back to the middle of the twentieth century, when its clear inhibitory action on the proteolytic activity of pepsin was demonstrated.

In addition, chondroitin sulfate performs numerous and essential functions, in the physiological sense, in those tissues and organs in which it is present, for example regulating the strength and elasticity of tissues and participating in the organisation of cytoskeletal structures. In therapy, said active principle is used in particular for treating various pathological conditions of cartilaginous joints, such as osteoarthrotic and inflammatory processes, and can also perform a protective action on cartilaginous tissue and sinovial fluid (Pipitone V.R., Drugs Explt., Clin. Res., XVII (1), 3, 1991; Rovetta G., Drugs Explt., Clin. Res., XVII (1), 53, 1991).

To be emphasised is the use of said active principle for treating arthrosis, since it is regarded as being able to protect and reconstitute articular cartilage damaged by degenerative phenomena, and of whose structure by its biochemical nature it is a fundamental component.

To achieve the desired therapeutic effects, the product is commonly used in the sodium salt form, salified at the various acid functions of the repeating disaccharide units, i.e. either at the carboxyl of the glucuronic acid or the sulfonic radicals of the acetylgalactosamine, and can be administered by various means, for example orally or parenterally, in its pure form or in the form of pharmaceutical compositions. For the purposes of the present invention also, sodium chondroitin sulfate is particularly preferred.

In general chondroitin sulfate, and therefore also sodium chondroitin sulfate, is poorly soluble in water due to its polymeric nature, with a tendency to form lumps which hydrate extremely slowly; in therapy, said active principle is mostly administered by oral means with daily doses ranging from 400 to 1200 mg for therapeutic periods lasting for months.

In the state of the art some pharmaceutical compositions containing sodium chondroitin sulfate have hitherto been known for oral use, in particular in the form of tablets, capsules and granulated active principle contained in sachets for suspending in water prior to administration. A further pharmaceutical form employed is an oral gel, available in special sealed sachets to be opened at the time of consumption. Oral gels comprising sodium chondroitin sulfates are disclosed in EP 0704216.

Given the high daily doses required for arthrosis treatment and the protracted administration periods (often the therapy has to be applied for the rest of the patient's life), known administration forms are problematic from the viewpoint of the patient's convenience (or "patient compliance") which must be as high as possible so as not to discourage the patient from correct and prolonged self-administration.

As regards capsules and tablets, it is apparent that the mere quantity of active principle required to formulate a daily dose creates a problem with the size of the resulting pharmaceutical forms. While capsules with contents exceeding one gram are no longer swallowable, the same is true for the relative tablets in which the problem of excessive size is still more accentuated by the fact that the compressibility required for tablet manufacture is attained by the addition of suitable excipients ("tableting aids"). In the case of the sachets containing granular active principle, self-administration of the daily chondroitin sulfate dose is made uncomfortable by the need to suspend the contents in water and the difficulty in removing lumps. Although this problem is partly relieved by the sachets containing chondroitin sulfate in gel form, self-administration still remains problematic. In fact, the gel must be "sucked" into the mouth by the patient directly from the sachet and so must possess a relatively low viscosity to enable the sachet to be completely emptied without needing to squeeze it with the hands, risking leakage of the contents. On the other hand, the low viscosity requires the use of totally waterproof sachets, openable without losses occurring. Sachets currently used for so-called "orogels", which reduce markedly the risk of spillage by the patient, impact considerably on the cost of the preparation. In fact, said sachets must also be reasonably protected against accidental puncture, since, given the required daily consumption, the patient is obliged to keep the sachets to hand, for example while travelling.

An object of the present invention is therefore the provision of a new formulation comprising the daily dose of chondroitin sulfate required for arthrosis therapy, demonstrating improved patient compliance compared to other known administration forms.

A further object of the present invention is the provision of a new formulation comprising chondroitin sulfate, the said formulation allowing for the realization of a new therapeutic application, discovered by the inventors of the present application.

Preferably the new chondroitin sulfate containing formulation of the present invention has an improved patient compliance and enables a twofold therapeutic action to be developed.

### SUMMARY

These and other objects which will be more apparent hereinafter are attained by a new chewable solid formulation, in which the chondroitin is already in hydrated form and dissolved in the medium. It concerns a chewable solid non-compressed pharmaceutical formulation for oral administration, comprising a chondroitin sulfate salt in a therapeutically effective quantity in an intimate mixture with, per gram of incorporated chondroitin sulfate salt, 500-1500 mg water, 25-3000 mg of at least one primary structuring agent chosen from the group consisting of: gum arabic, pectin, animal or vegetarian gelatin or a mixture thereof and 1500-8000 mg of at least one secondary structuring agent chosen from the group consisting of : soluble starches and agar-agar, sucrose, trehalose, glucose, isomalt, polyols, xylitol, maltitol, erythritol, maltodextrins, fructose or mixtures thereof, wherein the final formulation contains 7%-20% by weight of water.

Optionally, the new chewable solid formulations can contain, in lower amounts, preferably in quantities of 1-2000 mg per g of incorporated chondroitin sulfate salt, other excipients such as pharmaceutically acceptable softening agents, emulsifiers, pH regulators and/or flavourings and/or preservatives. Optionally, the new chewable solid formulations can contain further active principles required for arthrosis therapy, such as sodium glucosamine sulfate. In this case, the amount of further active principles provided, partially replaces the quantity of chondroitin sulfate in the formulations of the present application.

### BRIEF DESCRIPTION OF THE FIGURES.

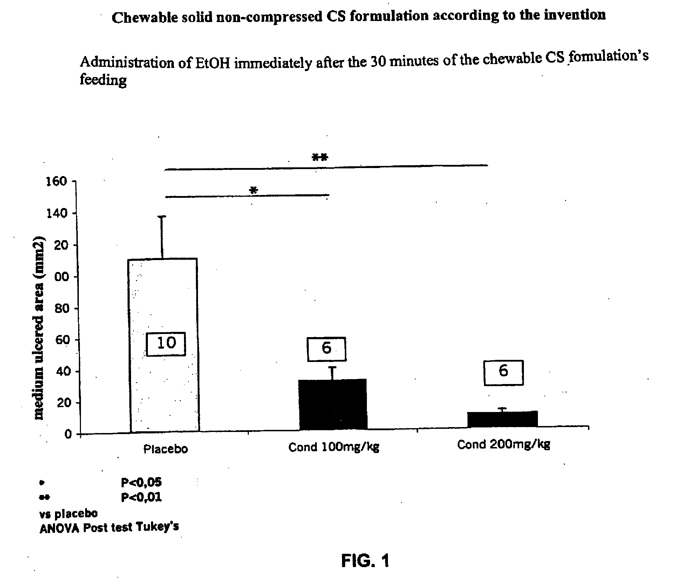

Figure 1 is the graphical representation of the medium ulcered area (Y-axis) induced in a test animal's stomach through ethanol challenge immediately after the administration (during a prior feeding time of 30 minutes) of different dosages (X-axis) of the new CS formulations according to the invention.

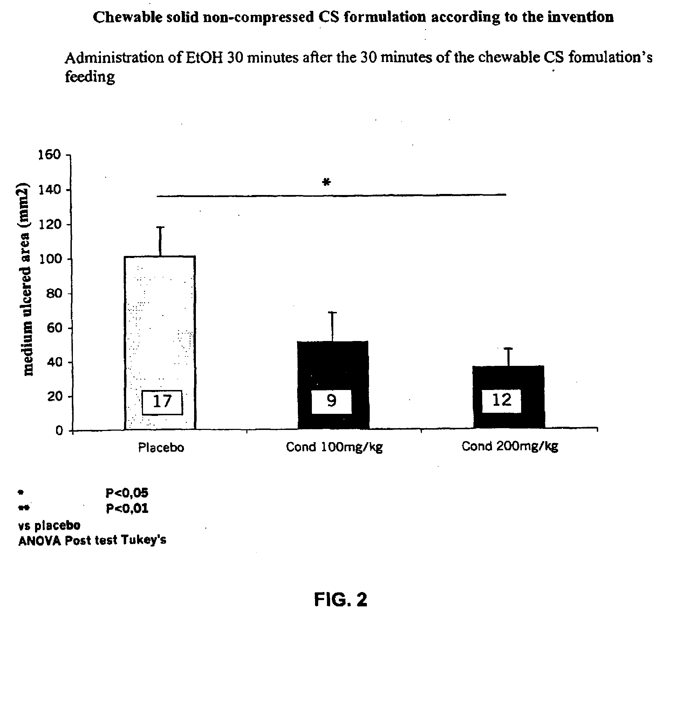

Figure 2 is the graphical representation of the medium ulcered area (Y-axis) induced in a test animal's stomach through ethanol challenge 30 minutes after the administration (during a prior feeding time of 30 minutes) of different dosages (X-axis) of the new CS formulations according to the invention.

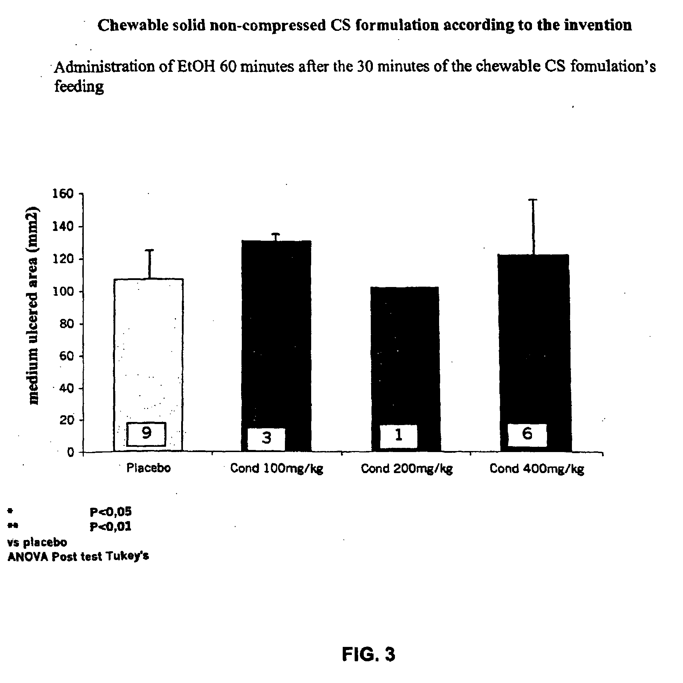

Figure 3 is the graphical representation of the medium ulcered area (Y-axis) induced in a test animal's stomach through ethanol challenge 60 minutes after the administration (during a prior feeding time of 30 minutes) of different dosages (X-axis) of the new CS formulations according to the invention.

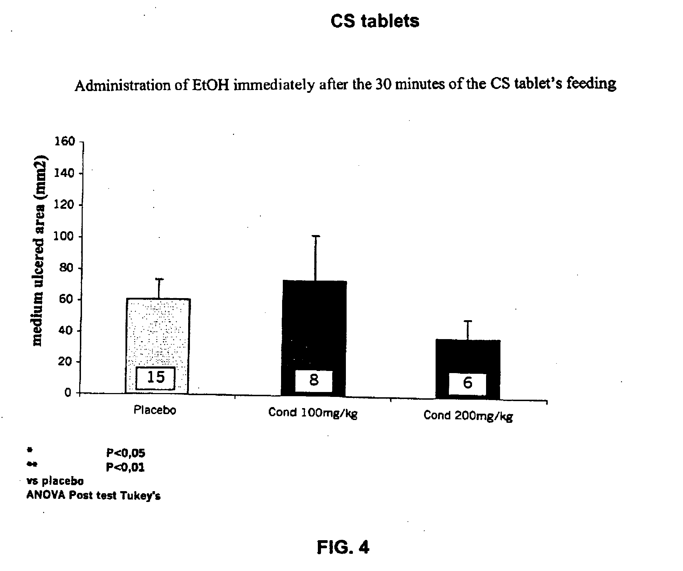

Figure 4 is the graphical representation of the medium ulcered area (Y-axis) induced in a test animal's stomach through ethanol challenge immediately after the administration (during a prior feeding time of 30 minutes) of different dosages (X-axis) of CS tablets.

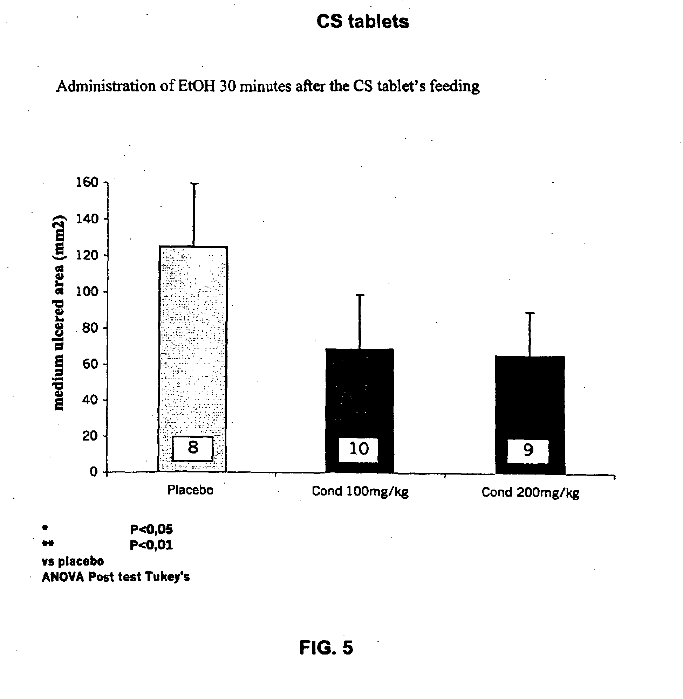

Figure 5 is the graphical representation of the medium ulcered area (Y-axis) induced in a test animal's stomach through ethanol challenge 30 minutes after the administration (during a prior feeding time of 30 minutes) of different dosages (X-axis) of the new CS formulations according to the invention.

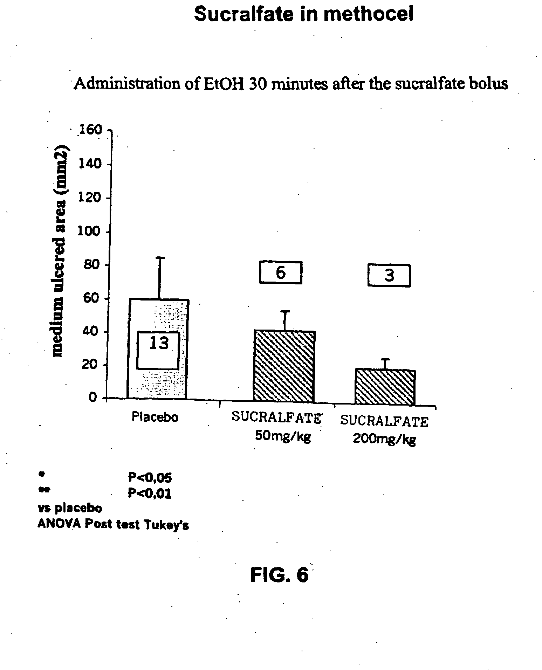

Figure 6 is the graphical representation of the medium ulcered area (Y-axis) induced in a test animal's stomach through ethanol challenge 30 minutes after the administration of different dosages (X-axis) of an intragastric bolus (all-at-once delivery *in situ*) of a commercial sucralfate preparation.

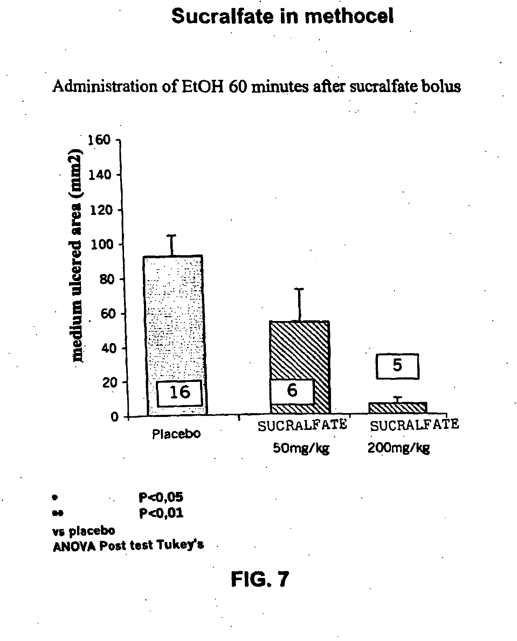

Figure 7 is the graphical representation of the medium ulcered area (Y-axis) induced in a test animal's stomach through ethanol challenge 60 minutes after the administration of different dosages (X-axis) of an intragastric bolus (all-at-once delivery *in situ*) of a commercial sucralfate preparation.

### DETAILED DESCRIPTION OF THE INVENTION.

The inventors of the present application have discovered that chondroitin sulfate, if administered in a suitable form, can exhibit gastroprotective characteristics. Therefore, an aspect of the present invention is the development of a new pharmaceutical composition which allows the gastroprotective qualities of chondroitin sulfate, and preferably those of sodium chondroitin sulfate, to be utilized.

Said application is particularly interesting in that classical gastroprotective formulations comprise active principles such as antacids (aluminium hydroxide, magnesium hydroxide), often in combination with simethicone which counteracts gas development. Said antacid formulations -unlike so called H2-blockers which instead are highly specific drugs aimed at suppressing gastric acid secretion-have a strictly symptomatic effect, but are prescribed on a large scale as they are considered safe and have been long accepted, at least as a coadjuvant, for the therapy of various digestive disturbances such as ulcus duodeni, ulcus ventriculi, gastric acidity, oesophageal reflux, NSAID-gastropathy, gastritis etc.

On the other hand, prolonged use of antacids is associated with the following limitations which affect their usefulness:
- If taken on an empty stomach antacids neutralize acid for a brief period only.
- To achieve a constant therapeutic activity over time, antacids must therefore be consumed not only close to meals but also during the night. If administered several times a day, the cost of antacid therapy can exceed that of daily consumption of an H2-blocker.
- Antacids interfere with many other drugs; due to their pH-modifying action, they interfere with the assimilation of other substances. Known examples of drugs susceptible to interactions with antacids are: benzodiazepine, captopril, steroids, flecainide, anti-ulcer drugs, phenytoin type drugs, iron, ketoconazole, lecodope, penicillamine, phenothiazine, quinidine, aspirin, salicylates, anti-diabetes drugs, tetracycline, ticlopidine and valproic acid.

At least one hour must therefore be left between consumption of antacids and consumption of the aforementioned drugs, a requirement which can become tiresome in prolonged therapies. In this context the interaction between antacids and, often simultaneously prescribed, non-symptomatic anti-ulcer drugs such as ranitidine (Zantac), a classic H2-blocker, is particularly precarious.

Consequently, the need still remains for the development of gastroprotective formulations which do not interact with other drugs and can replace at least one of the frequent consumptions taken every daily envisaged in prolonged therapy with "antacids" of the known art.

The chondroitin sulfate formulations of the present invention, in contrast with known formulations, have been developed to impart a gastroprotective activity to the ingested chondroitin sulfate, thus enabling it to have a twofold therapeutic action: a gastroprotective one, and the classical one of arthrosis therapy. Given that there is no evidence that gastroprotective activity would be associated with side effects of the administered chondroitin, consumption of the formulations of the present invention is not only indicated for subjects needing both therapies, but also for subjects who intend dealing only with arthrosis. This is because the formulation of the present invention is also advantageous in terms of patient compliance, i.e. it allows for the provision of daily dosage units which can comprise even up to 1200 mg of active principle without requiring, at the time of ingestion, the preparation of aqueous suspensions by the patient or the packaging, by the pharmaceutical manufacturing company, of semi-liquid preparations (orogel) in special sachets, with particular emptying characteristics, which then have to act as dispensers at the time of consumption by the patient.

In particular, the new formulations of the present invention include the following advantages:
- They do not require dilution water prior to being swallowed.
- They do not require the use of glasses or spoons on swallowing.
- They are characterised by being very pleasant to take due to the active principle being well masked.
- They provide a longer time in contact with the gastric surface of the patient.
- They allow, in comparison with traditional non-dispersed solid forms (tablets, capsules), a greater amount per single dose without lowering patient compliance.

These factors are important when it is considered that known administration forms were sachets comprising 400 or 800 mg, tablets comprising 400 or 800 mg, capsules comprising 400 mg or "orogel" sachets comprising 400, 800 or 1200 mg of active principle. Instead, the new chewable tablets of 1.33 and 1.79 g described in Khan et al. ("Formulation Development and Stability Evaluation of a Multicomponent Nutritional Supplement", Pharmaceutical Technology, 25(4), 38-48 (2001)) can contain (400 mg of chondroitin sulfate and 500 mg of glucosamine HCl) to a maximum of 900 mg of active principle, but are not suitable for allowing a prolonged contact between the active principle and gastric surface of the patient.

As previously stated, the new chewable solid formulations of the present invention comprise chondroitin sulfate as active principle, preferably sodium chondroitin sulfate, in a therapeutically effective quantity, in an intimate mixture with, per gram of incorporated chondroitin sulfate salt, 500-1500 mg water, 25-3000 mg of at least one primary structuring agent chosen from the group consisting of: gum arabic, pectin, animal or vegetarian gelatin or a mixture thereof and 1500-8000 mg of at least one secondary structuring agent chosen from the group consisting of : soluble starches and agar-agar, sucrose, trehalose, glucose, isomalt, polyols, xylitol, maltitol, erythritol, maltodextrins, fructose or mixtures thereof, wherein the final formulation contains 7%-20% by weight of water.

In particular the inventors of the present invention have found that incorporating primary structuring agents such as gum arabic, pectin and/or animal and/or vegetarian gelatin, together with other structuring agents, enables chewable, solid pharmaceutical forms to be obtained, formulations which can be produced without the use of compression force. Excluding the compression force from the production cycle is important, as the dies required for tablet manufacture are only available in a certain size, at least in the standard model. Moreover, tablet production would require limiting the amount of water in the formulation to very low percentages, thus excluding the possibility of pre-dissolving the active principle. Very dry formulations, such as chewable tablets, are unpleasant for the patient who tends to swallow rapidly the lumps produced by chewing. Predissolving the active principle together with its incorporation into the structuring agents are important to provide the gastroprotective quality of the formulation. In fact, dissolution in the mouth and the presence of structuring agents which regulate the release bring about an extended and measured conveyance of the active principle - already pre-dissolved in the aqueous matrix of the formulation and then further and uniformly softened in the mouth by contact with saliva, then swallowed - , to arrive at the gastric mucosa where it can better perform its gastroprotective properties. This does not happen with traditional "orogel" consumption even though it is also partially pre-dissolved, since the sensation (or "feel") of the gel in the mouth is not immediately perceived as pleasant by the patient who tends to swallow the whole dose at once. The gel therefore arrives rapidly into the stomach and leaves it rapidly, being very fluid. The necessary contact time with the gastric mucosa, which is important for the activity, is therefore absent. In fact, it can be seen that due to the considerable difference in viscosity between the orogels and the stomach contents and due to the start of solvation at the gel surface (with consequent formation of a surface "film" which envelops the entire portion), the orogels tend to pass through the gastric environment without substantially mixing with it.

In the formulation found by the inventors of the present invention, the primary structuring agents such as gum arabic, pectin and animal or vegetarian gelatin or their mixtures, enable a pre-dissolved preparation of chondroitin sulfate in water to be sufficiently stabilized to form an essentially solid matrix which incorporates the active principle. In performing this function, these structuring agents are assisted on the one hand by the chondroitin sulfate itself which acts as a co-structuring agent (thus limiting, to a certain extent, active principle dilution in the pharmaceutical form) and on the other hand by the secondary structuring agents which are chosen from the group consisting of: soluble starches and agar-agar, sucrose, glucose, isomalt, polyols, xylitol, maltitol, erythritol, trehalose, maltodextrins and fructose or mixtures thereof.

It is important to emphasise that the role of the secondary structuring agents, in addition to contributing to the solidification of the final pharmaceutical form already referred to, lies also in the controlled disintegration of the final matrix by saliva as it is softened in the mouth. In fact the secondary structuring agents are characterised by their greater solubility in saliva leading to their migration from the matrix thus increasing its surface, useful for slowing the availability of the active principle in the gastric mucosa. Consequently, the disintegration in the mouth of the new chewable solid pharmaceutical formulation, allows further solvation of the active principle which is released in a controlled manner from the matrix at the moment of its decomposition in the mouth of the patient.

The so-called additional excipients are preferred but not necessary for improving, at the moment of administration, the sensation produced in the mouth of the patient by the formulations of the present invention. They can therefore benefit patient compliance. Among these additional excipients are pharmaceutically acceptable softening agents that can be chosen on the one hand from any pharmaceutically acceptable liquid or solid, vegetable or animal fat, e.g. cocoa butter, seed oil, palm oil or coconut oil etc or on the other hand from softening agents chosen from pharmaceutically acceptable alcohols, such as glycerin. Other additional excipients are emulsifiers such as lecithin or glyceryl monostearate. Other additional excipients are acidity regulators, these also being chosen from all known pharmaceutically acceptable ones, e.g. sodium carbonate, citric acid or others. Other additional excipients are flavourings, these also being chosen from all known pharmaceutically acceptable ones whether natural or synthetic, for example vanilla flavour. Other additional excipients are preservatives, these also being chosen from all known pharmaceutically acceptable ones, e.g. ascorbic or benzoic acid.

The new formulations of the present invention can be obtained by processes which comprise mixing chondroitin sulfate with water or with solutions or dispersions of the additional excipients, including structuring agents, to promote pre-dissolution, preferably at a high temperature, optionally followed by drying of the mix thus obtained until the water content is that intended in the finished formulations. Preferably, the new formulations of the present invention are provided in single units comprising the therapeutically required daily intake of chondroitin sulfate, said units weighing between 500 mg and 8 g. Given the high patient compliance guaranteed by the formulations of the present invention, single units comprising half of the therapeutically necessary daily dosage of chondroitin sulfate, can also be provided, said units weighing from 500 mg to 6 g.

### EXPERIMENTAL PART

### EXAMPLES

### Example 1: Preparation of chewable solid pharmaceutical formulation containing sodium chondroitin sulfate.

103.35 g of isomalt are melted at 130°C in a steel container while stirring manually; the temperature is then brought to 90-100°C, the heat is reduced and 150 g of glucose syrup, 2 g of pectin, 45 g of sodium chondroitin sulfate, 0.45 g of acesulfame potassium, 0.6 g of caramel flavouring and 0.6 g of lemon flavouring are added, maintaining the temperature between 90 and 100°C. The mass, maintained as a fluid, is dispensed into suitable starch moulds, comprising special shapes which are suited to receiving single portions of the mass originating from the dispenser. It is then left to stabilize for 24 hours in an oven at 30°C and 12 to 15% humidity.

Preparations were thus obtained having an average weight of 4.5 g and containing 0.8 g of sodium chondroitin sulfate per unit. Using the same method, single units of 6.75 g can be obtained comprising 1.2 g sodium chondroitin sulfate by adjusting the dispenser and using different moulds with larger capacity of shapes.

### Example 2: Preparation of chewable solid pharmaceutical formulation containing chondroitin sulfate.

In a steel container jacketed for steam heating, and with mechanical stirrer, 50.19 kg of water are brought to 80°C then 1.5 kg of pectin and 55.31 kg of gum arabic are added. The entirety is stirred while maintaining the temperature at 80°C for 60 minutes until it dissolves; the solution is then filtered using a 200 micron panel filter. Separately, 56.72 kg of 75% maltitol are concentrated until a thick mass is obtained by heating at 120°C for 40 minutes in a steel container jacketed for steam heating, and with mechanical stirring. 23.95 kg of sodium chondroitin sulfate are then added and a previously prepared solution of gum arabic is incorporated while maintaining the temperature at 120°C. The temperature is then lowered to 90°C and 4.5 kg of glycerol, 0.675 kg of citric acid, 75 g of acesulfame potassium, 0.3 kg of lemon flavouring and 0.28 kg of vanilla flavouring are incorporated.

The hot mass is dispensed at 90°C into the starch moulds so that an identical amount is dispensed into each mould. The dispensed mass is oven dried in the moulds at 50°C for 48 hours until residual humidity is 15%.

After removing the single doses from the moulds, formulations of 3.3 g average weight were obtained containing 0.45 g of sodium chondroitin sulfate per unit.

### Example 3: Preparation of chewable solid pharmaceutical formulations containing 0.4 g sodium chondroitin sulfate.

A solution is prepared containing 6.5 kg of sodium chondroitin sulfate, 0.4 kg of pectin, 0.022 kg of acesulfame potassium in 10.334 kg of water.

In a suitable cooker a mass formed from 12.69 kg of maltitol, 17.5 kg of isomalt, 0.258 kg of glyceryl monostearate, 2.6 kg of vegetable fats, 0.0305 kg of soya lecithin is cooked at 110°C for 40 minutes under vacuum, then maintained under vacuum for 10 minutes at -0.4 bar.

The mass is cooled to 90°C and to it the previously prepared solution containing the sodium chondroitin sulfate and pectin are added together with 0.0655 kg of caramel flavouring and 0.0655 kg of vanilla flavouring.

The resulting mass is poured onto a water cooled board bringing its temperature to 23-25°C.

The mixture is kneaded for 10 minutes to incorporate air. The mass is further cooled to 18°C, divided into portions and packaged using a cut and wrap machine.

Single units of 2.8 g were obtained equal to 0.4 g of sodium chondroitin sulfate. The final water content, without further drying, was 15% by weight.

### Example 4: Preparation of chewable solid pharmaceutical formulations containing 0.8 g chondroitin sulfate.

Formulations are prepared as described in example 3, but in the final cutting and wrapping step the machine is adjusted to obtain 5.6 g units equal to 0.8 g of active principle. The final water content, without further drying, was 15% by weight.

### Example 5: Preparation of chewable solid pharmaceutical formulations containing sodium chondroitin sulfate and glucosamine hydrochoride.

Formulations are prepared as described in example 1, but 22.5 kg of sodium chondroitin sulfate and 22.5 kg of glucosamine hydrochloride are added to the mass.

Formulations in 4.5 g units equal to 0.4 g of sodium chondroitin sulfate and 0.4 g glucosamine hydrochloride were obtained. Following oven drying, the final moisture content amounts to 13% by weight.

### Example 6: Preparation of chewable solid pharmaceutical formulations containing sodium chondroitin sulfate and sodium glucosamine sulfate.

Formulations as described in example 1 are prepared, but 22.5 kg of chondroitin sulfate and 22.5 kg of sodium glucosamine sulfate are added to the mass.

Formulations in 4.5 g units equal to 0.4 g of sodium chondroitin sulfate and 0.4 g sodium glucosamine sulfate were obtained. Following oven drying, the final moisture content amounts to 12% by weight.

### Example 7: Preparation of chewable solid pharmaceutical formulations containing sodium chondroitin sulfate and glucosamine hydrochoride.

Formulations as described in example 2 are prepared, but 12 kg of sodium chondroitin sulfate and 12 kg of glucosamine hydrochloride are added to the mass.

Formulations in 4.4 g units equal to 0.3 g of sodium chondroitin sulfate and 0.3 g glucosamine hydrochloride were obtained. Following oven drying, the final moisture content amounts to 13% by weight.

### Example 8: Preparation of formulations containing sodium chondroitin sulfate and sodium glucosamine sulfate.

Formulations as described in example 2 are prepared, but 12 kg of sodium chondroitin sulfate and 12 kg of sodium glucosamine sulfate were added to the mass.

Formulations in 4.4 g units equal to 0.3 g of chondroitin sulfate and 0.3 g sodium glucosamine sulfate were obtained. Following oven drying, the final moisture content amounts to 14% by weight.

### Example 9: Preparation of chewable solid pharmaceutical formulations containing sodium chondroitin sulfate and glucosamine hydrochoride.

Formulations as described in example 3 are prepared, but 3.25 kg of chondroitin sulfate and 3.25 kg of glucosamine hydrochloride were added to the mass.

Formulations of 5.7 g equal to 0.4 g of chondroitin sulfate and 0.4 g glucosamine hydrochloride were obtained. Following oven drying, the final moisture content amounts to 16% by weight.

### Example 10: Preparation of formulations containing sodium chondroitin sulfate and sodium glucosamine sulfate.

Formulations as described in example 3 are prepared, but 3.25 kg of chondroitin and 3.25 kg of sodium glucosamine sulfate are added to the mass.

Formulations of 5.7 g equal to 0.4 g of chondroitin sulfate and 0.4 g sodium glucosamine sulfate were obtained. Without further drying, the final moisture content amounts to 15% by weight.

### Example 11: Preparation of chondroitin sulfate containing 0.39 g of sodium chondroitin sulfate with sucrose and glucose.

A solution containing 390 g of sodium chondroitin sulfate, 20 g of pectin in 1 kg of water is prepared.

In a suitable vacuum cooker a mass formed of 1.5 kg of sucrose and 1 kg of glucose syrup, 24.6 g of glyceryl monostearate, 250 g of vegetable fats, 3 g of soya lecithin is cooked at 110° for 40 minutes and is then maintained under vacuum for 10 minutes at -0.4 Bar.

While cooling, an additional 500 g of sucrose are added, the mass is cooled to 90° and the previously prepared solution containing sodium chondroitin sulfate and pectin is added together with 6 g of caramel flavouring and 6 g of vanilla flavouring.

The resulting mass is poured onto a water cooled board bringing down its temperature to 23-25°C.

The mixture is kneaded for 10 minutes to incorporate air. The mass, further cooled to 18°C, is packaged using a cut and wrap machine.

Formulations of 4.2 g equal to 0.39 g of sodium chondroitin sulfate were obtained having a residual moisture content of 12% by weight.

### Example 12: Preparation of formulations containing sodium chondroitin sulfate and glucosamine hydrochloride in sucrose and glucose.

Formulations as described in example 11 were prepared, but 2 kg of sucrose and glucose, 265 g of chondroitin sulfate and 125 g of glucosamine hydrochloride were added to the mass.

Formulations were obtained which were then subdivided into units of 3 g equal to 0.265 g of chondroitin sulfate and 0.125 g glucosamine hydrochloride. Residual moisture content: 12% by weight.

### Example 13: Preparation of formulations containing sodium chondroitin sulfate and sodium glucosamine sulfate in sucrose and glucose.

Formulations as described in example 11 were prepared, but 2 kg of sucrose and glucose; 265 g of chondroitin sulfate and 125 g of sodium glucosamine sulfate were added to the mass.

Formulations were obtained which were then subdivided into units of 3 g equal to 0.265 g of chondroitin sulfate and 0.125 g sodium glucosamine sulfate. Residual moisture content: 12% by weight.

### Example 14: Investigation and comparative testing of the gastroprotective properties of the novel CS formulations provided by the present invention.

The aim of this investigation was to compare the gastroprotective effects of orally administered chondroitin sulfate formulations (chewable solid non-compressed formulations as herein described vs. tablets) on ethanol induced acute gastric ulcers in rats and to compare these in turn with the *in situ* application of the conventional gastroprotective agent sucralfate. In addition, the dose-dependency and duration of said gastroprotective effect were considered.

### Materials and methods

### Substances

Chondroitin sulfate supplied by the firm IBSA in the following formulations:
• Chondroitin sulfate in chewable, solid non-compressed formulation as herein described, available at
40 mg/400 mg corresponding to administration of 100 mg/kg, 200 mg/kg and 400 mg/kg (with half, single and double quantities of chewable, solid non-compressed formulation as herein described).
The actually administered formulation were as follows:

| | |
|---|---|
| sodium chondroitin sulfate: | 10% |
| pectin: | 1% |
| glucose | 40.31% |
| glyceryl monostearate | 0.78% |
| vegetarian fats | 7.86% |
| soja lecitihin | 0.10% |
| sucrose | 28.55% |
| water | 11% |
| caramel flavour | 0.2% |
| vanilla flavour | 0.2% |

• Placebo chewable, solid non-compressed formulation as herein described

| | |
|---|---|
| pectin: | 1% |
| glucose | 50.31% |
| glyceryl monostearate | 0.78% |
| vegetarian fats | 7.86% |
| soja lecitihin | 0.10% |
| sucrose | 28.55% |
| water | 11% |
| caramel flavour | 0.2% |
| vanilla flavour | 0.2% |

• Chondroitin sulfate tablets available in two dosages:
20 mg/400 mg corresponding to administration of 100 mg/kg
40 mg/400 mg corresponding to administration of 200 mg/kg
The formulations of the tablets were as follows:

| | |
|---|---|
| Sodium chondroitin sulphate | 5% |
| Hydroxypropylmethyl cellulose | 46.25% |
| Microcrystalline cellulose | 48.66% |
| Magnesium stearate | 0.0885% |

| | |
|---|---|
| Sodium chondroitin sulphate | 10% |
| Hydroxypropylmethyl cellulose | 46.25% |
| Microcrystalline cellulose | 43.66% |
| Magnesium stearate | 0.0885% |

• Placebo tablets

| | |
|---|---|
| Hydroxypropylmethyl cellulose | 46.25% |
| Microcrystalline cellulose | 53.66% |
| Magnesium stearate | 0.0885% |

Sucralfate produced by Merck Generics:
- Sucralfate suspension in methocel available at a concentration of 87 mg/ml. Precise aliquots were administered in a final volume of 2.3 ml per kg, to give doses of 50 mg/kg and 200 mg/kg
- Placebo methocel carrier

The chondroitin sulfate was administered orally in the form of the chewable solid non-compressed formulations as herein described during a feeding time of 30 minutes in doses of 100 and 200 mg/kg, where after, namely at 0, 30 or 60 minutes following the feeding period, intragastric administration of ethanol was triggered. The same protocol (feeding time of 30 minutes) was adopted for the CS tablets, with intragastric ethanol challenge at 0 and 30 minutes following administration. Placebo chewable solid non-compressed formulations as herein described and tablets were likewise administered to the control groups.

The sucralfate and the respective placebo were administered by intragastric probe and the intragastric administration of ethanol was carried out 30 and 60 minutes thereafter.

On top of that, a 400 mg/kg dose was evaluated for chondroitin sulfate in chewable solid non-compressed formulations as herein described, given 60 minutes before the administration of ethanol.

### Ethanol 90%, formalin 4% (Sigma)

### Animals

In the experiment female Wistar rats weighing 180-200 g were used having fasted for the 12 hours preceding the experiment and having free access to water. The experiment was authorised by the ethical Committee and by the competent ministerial authority and conducted observing the current regulations relating to animal experimentation (DL 116/92).

### Experimental protocol

After being randomly assigned to different treatment groups, the animals were separated into individual cages. Each animal was given a sufficient quantity of chewable solid non-compressed formulations as herein described or tablets to enable 100 or 200 mg//kg of chondroitin sulfate to be consumed in the space of 30 minutes. The sucralfate suspension was administered by an intragastric probe in the form of a bolus and in quantities sufficient to give doses of 50 mg/kg and 200mg/kg. The control group received the corresponding placebo preparations.

After 30, 60 or 90 minutes from the start of the treatment as a whole (i.e. comprising the feeding time) 1 ml of 90% ethanol was administered intragastrically. After 1 hour the animals were killed under anaesthetic ether by carbon dioxide, the stomachs were tied at the cardias and pylorus, filled with 6 ml of 4% formalin, explanted and immersed in a 4% formalin solution. Subsequently the stomachs were opened by making a cut along the greater curvature, stretched between two transparent supports and their image scanned onto a computer.

The gastric damage caused by oral administration of ethanol was evaluated by measuring the ulcerated area using the image processing and analysis programme, Scion Image 1.62 (Haseeb Ahmad Khan, Journal of Pharmacological and Toxicological Methods 49 (2004) 89-95) by an experimenter without knowledge of the treatment.

The results, expressed in terms of mm² of ulcerated area, were given as means ± standard error and analysed statistically by applying the Anova test followed by the Tuckey test. *P<0.05 and **P<0.01 indicate significant or highly significant differences.

### Results

In the stomachs of rats belonging to the control groups treated with placebo and subsequently exposed to ethanol abuse, the presence of extensive areas with haemorrhagic erosions was observed which were quantified by computer analysis and expressed as average ulcerated area (mm²) (see Figures 1-7).

Consumption of chondroitin sulfate in the form of the novel chewable solid non-compressed formulations as herein described protected the gastric mucosa from the necrotising action of ethanol in a dose and time dependent manner (Figures 1-2).

The greatest reduction in gastrolesive effects of ethanol was observed when the aggressive agent was administered 30 minutes after the start of chondroitin sulfate feeding (Figure 1). In this case, both the chondroitin sulfate doses proved to be gastroprotective. The protective effect still remained significant during the next 30 minutes, though this was more evident in the case of consumption of the higher dose of chondroitin sulfate (200 mg/kg) in the novel chewable solid non-compressed formulations as herein described (Figure 2). Effectiveness of gastroprotection by the chondroitin sulfate as chewable solid non-compressed formulations as herein described is however reduced if ethanol administration is further delayed. Such reduction of effectiveness is observed even when the chondroitin sulfate dose is doubled to 400 mg/kg (Figure 3).

On the other hand, consumption of the same chondroitin sulfate doses, formulated as tablets, while demonstrating a tendency to reduce the ulcerated area, did not provide significant protection of the gastric mucosa against ethanol, independently of the moment of administering the necrotising agent (Figures 4 and 5).

Moreover, it can be seen that the gastroprotective effects recorded with chondroitin sulfate 200 mg/kg in the chewable solid non-compressed formulation as herein described is comparable to the one observed following intragastric bolus administration of a 200 mg/kg sucralfate suspension in methocel (Figures 6 and 7).

## Claims

1. A chewable solid non-compressed pharmaceutical formulation for oral administration, comprising a chondroitin sulfate salt in a therapeutically effective quantity, in an intimate mixture with, per gram of incorporated chondroitin sulfate salt, 500-1500 mg water, 25-3000 mg of at least one primary structuring agent chosen from the group consisting of: gum arabic, pectin, animal or vegetarian gelatin or a mixture thereof and 1500-8000 mg of at least one secondary structuring agent chosen from the group consisting of : soluble starches and agar-agar, sucrose, trehalose, glucose, isomalt, polyols, xylitol, maltitol, erythritol, maltodextrins, fructose or mixtures thereof, wherein the final formulation contains 7%-20% by weight of water.

2. Pharmaceutical formulation as claimed in the first claim, wherein the chondroitin sulfate salt is partially replaced, preferably from 1% to 60%, with other active principles effective in arthrosis therapy, for example glucosamine.

3. Pharmaceutical formulation as claimed in claim 1 or 2, wherein the chondroitin sulfate salt is sodium chondroitin sulfate.

4. Pharmaceutical formulation as claimed in claim 2 or 3, wherein the glucosamine is chosen from the group consisting of glucosamine hydrochloride and sodium glucosamine sulfate.

5. Pharmaceutical formulation as claimed in one or more of the preceding claims, comprising, per gram of incorporated chondroitin sulfate salt, 1-2000 mg of other excipients chosen from the group consisting of pharmaceutically acceptable softening agents, pharmaceutically acceptable emulsifiers, pH regulators and/or flavourings and/or preservatives, or mixtures thereof.

6. Formulation as claimed in one or more of the preceding claims, **characterised in that** the final formulation contains 9%-17% of water by weight.

7. Formulation as claimed in one of the preceding claims, **characterised in that** the primary structuring agent is present, per gram of incorporated chondroitin sulfate, in a quantity of 30-1000mg, and the secondary structuring agent is present, per gram of incorporated chondroitin sulfate, in a quantity of 2500-8000 mg.

8. Formulation as claimed in claim 7, wherein the additional excipients consist of pH regulators, and/or flavourings and/or preservatives or their mixtures, said excipients being present, per gram of incorporated chondroitin sulfate, in a total quantity of 1-100 mg.

9. Formulation as claimed in claim 8, wherein the additional excipients in addition to the pH regulator, and/or flavouring and/or preservative, comprise pharmaceutically acceptable softening agents, said pharmaceutically acceptable softening agents being present, per gram of incorporated chondroitin sulfate, in a quantity of 100-800 mg.

10. Formulation as claimed in one or more of claims 1-6, **characterised in that** the primary structuring agent is present, per gram of incorporated chondroitin sulfate, in a quantity of 1001-3000 mg, and the secondary structuring agent is present, per gram of incorporated chondroitin sulfate, in a quantity of 1500-2499 mg.

11. Formulation as claimed in claim 10, wherein the additional excipients consist of pharmaceutically acceptable softening agents and/or flavourings and/or structuring agents, said additional excipients being present, per gram of incorporated chondroitin sulfate, in a quantity of 30-800 mg.

12. Process for obtaining the formulations of one or more of the preceding claims, comprising mixing chondroitin sulfate with water or with solutions or dispersions of the additional excipients, including structuring agents, to promote pre-dissolution, preferably at a high temperature, optionally followed by drying of the mixture thus obtained, until the water content is that provided for the final formulations.

## Patentansprüche

1. Kaubare, feste, nicht-komprimierte pharmazeutische Formulierung zur oralen Verabreichung, umfassend ein Chondroitinsulfatsalz in einer therapeutisch wirksamen Menge, in einer intensiven Vermischung mit, pro Gramm von enthaltenem Chondroitinsulfatsalz, 500-1500 mg Wasser, 25-3000 mg von wenigstens einem primären Strukturmittel, ausgewählt aus der Gruppe bestehend aus: Gummiarabikum, Pektin, tierischer oder pflanzlicher Gelatine oder einer Mischung daraus, und 1500-8000 mg von wenigstens einem sekundären Strukturmittel, ausgewählt aus der Gruppe bestehend aus: löslichen Stärken und Agar-Agar, Saccharose, Trehalose, Glucose, Isomalt, Polyolen, Xylitol, Maltitol, Erythritol, Maltodextrinen, Fructose oder Mischungen davon, wobei die Endformulierung 7-20 Gew.-% Wasser enthält.

2. Pharmazeutische Formulierung wie im ersten Anpruch beansprucht, in der das Chondroitinsulfatsalz teilweise, bevorzugt von 1 % bis 60 %, durch andere aktive Grundbestandteile, die bei der Arthrosetherapie effektiv sind, zum Beispiel Glucosamin, ersetzt ist.

3. Pharmazeutische Formulierung wie in Anspruch 1 oder 2 beansprucht, in der das Chondroitinsulfatsalz Natriumchondroitinsulfat ist.

4. Pharmazeutische Formulierung wie in Anspruch 2 oder 3 beansprucht, in der das Glucosamin aus der Gruppe, bestehend aus Glucosaminhydrochlorid und Natriumglucosaminsulfat, ausgewählt ist.

5. Pharmazeutische Formulierung wie in einem oder mehreren der vorhergehenden Ansprüche beansprucht, umfassend, pro Gramm von enthaltenem Chondroitinsulfatsalz, 1-2000 mg andere Hilfsmittel, ausgewählt aus der Gruppe, bestehend aus pharmazeutisch zulässigen Weichmachern, pharmazeutisch zulässigen Emulgatoren, pH-Regulatoren und/oder Geschmacksstoffen und/oder Konservierungsstoffen oder Mischungen daraus.

6. Formulierung wie in einem oder mehreren der vorhergehenden Ansprüche beansprucht, **dadurch gekennzeichnet, dass** die Endformulierung 9-17 Gew.-% Wasser enthält.

7. Formulierung wie in einem der vorhergehenden Ansprüche beansprucht, **dadurch gekennzeichnet, dass** das primäre Strukturmittel, pro Gramm von enthaltenem Chondroitinsulfat, in einer Menge von 30-1000 mg vorhanden ist und dass das sekundäre Strukturmittel, pro Gramm von enthaltenem Chondroitinsulfat, in einer Menge von 2500-8000 mg vorhanden ist.

8. Formulierung wie in Anspruch 7 beansprucht, in der die zusätzlichen Hilfsmittel aus pH-Regulatoren und/oder Geschmacksstoffen und/oder Konservierungsstoffen oder deren Mischungen bestehen, wobei diese Hilfsstoffe, pro Gramm von enthaltenem Chondroitinsulfat, in einer Gesamtmenge von 1-100 mg vorhanden sind.

9. Formulierung wie in Anspruch 8 beansprucht, in der die zusätzlichen Hilfsmittel zusätzlich zu dem pH-Regulator und/oder Geschmacksstoff und/oder Konservierungsstoff pharmazeutisch zulässige Weichmacher umfassen, wobei diese pharmazeutisch zulässigen Hilfsstoffe, pro Gramm von enthaltenem Chondroitinsulfat, in einer Menge von 100-800 mg vorhanden sind.

10. Formulierung wie in einem oder mehreren der Ansprüche 1-6 beansprucht, **dadurch gekennzeichnet, dass** das primäre Strukturmittel, pro Gramm von enthaltenem Chondroitinsulfat, in einer Menge von 1001-3000 mg vorhanden ist und dass das sekundäre Strukturmittel, pro Gramm von enthaltenem Chondroitinsulfat, in einer Menge von 1500-2499 mg vorhanden ist.

11. Formulierung wie in Anspruch 10 beansprucht, in der die zusätzlichen Hilfsstoffe aus pharmazeutisch zulässigen Weichmachern und/oder Geschmacksstoffen und/oder Strukturmitteln bestehen, wobei diese zusätzlichen Hilfsstoffe, pro Gramm von enthaltenem Chondroitinsulfat, in einer Menge von 30-800 mg vorhanden sind.

12. Verfahren zur Herstellung der Formulierungen nach einem oder mehreren der vorhergehenden Ansprüche, umfassend das Mischen von Chondroitinsulfat mit Wasser oder mit Lösungen oder Dispersionen der zusätzlichen Hilfsstoffe, einschließlich Strukturmitteln, um eine Vorauflösung zu begünstigen, vorzugsweise bei einer hohen Temperatur, optional gefolgt von Trocknen der daraus erhaltenen Mischung, bis der Wassergehalt der für die Endformulierungen bereitgestellte ist.

## Revendications

1. Formulation pharmaceutique à mâcher solide, non comprimée pour administration orale, comprenant un sel de sulfate de chondroïtine en une quantité thérapeutiquement efficace, en mélange intime avec, par gramme de sel de sulfate de chondroïtine incorporé, de 500 à 1500 mg d'eau, de 25 à 3000 mg d'au moins un agent de texture primaire choisi dans le groupe constitué de: la gomme arabique, la pectine, la gélatine animale ou végétale ou un mélange de celles-ci et de 1500 à 1800 mg d'au moins un agent de texture secondaire choisi dans le groupe constitué de: des amidons solubles et l'agar-agar, le saccharose, le tréhalose, le glucose, l'isomalt, des polyols, le xylitol, le maltitol, l'érythritol, des maltodextrines, le fructose ou des mélanges de ceux-ci, où la formulation finale contient de 7 % à 20 % en poids d'eau.

2. Formulation pharmaceutique selon la première revendication, dans laquelle le sel de sulfate de chondroïtine est partiellement remplacé, de préférence de 1 % à 60 %, par d' autres principes actifs efficaces dans la thérapie de l'arthrose, par exemple la glucosamine.

3. Formulation pharmaceutique selon la revendication 1 ou 2, dans laquelle le sel de sulfate de chondroïtine est le sulfate de chondroïtine sodique.

4. Formulation pharmaceutique selon la revendication 2 ou 3, dans laquelle la glucosamine est choisie dans le groupe constitué de la glucosamine chlorhydrate et le sulfate de glucosamine sodique.

5. Formulation pharmaceutique selon une ou plusieurs des revendications précédentes, comprenant, par gramme de sel de sulfate de chondroïtine incorporé, de 1 à 2000 mg d'autres excipients choisis dans le groupe constitué d'agents adoucissants pharmaceutiquement acceptables, d'émulsifiants pharmaceutiquement acceptables, de régulateurs de pH et/ou d'arômes et/ou de conservateurs, ou des mélanges de ceux-ci.

6. Formulation pharmaceutique selon une ou plusieurs des revendications précédentes, **caractérisée en ce que** la formulation finale contient de 9 % à 17 % d'eau en poids.

7. Formulation selon l'une des revendications précédentes, **caractérisée en ce que** l'agent de texture primaire est présent, par gramme de sulfate de chondroïtine incorporé, en une quantité de 30 à 1000 mg, et l'agent de texture secondaire est présent, par gramme de sulfate de chondroïtine incorporé, en une quantité de 2500 à 8000 mg.

8. Formulation selon la revendication 7, dans laquelle les excipients supplémentaires sont constitués de régulateurs de pH et/ou d'arômes et/ou de conservateurs ou leurs mélanges, lesdits excipients étant présents, par gramme de sulfate de chondroïtine incorporé, en une quantité totale de 1 à 100 mg.

9. Formulation selon la revendication 8, dans laquelle les excipients supplémentaires en plus du régulateur de pH, et/ou des arômes et/ou des conservateurs, comprennent des agents adoucissants pharmaceutiquement acceptables, lesdits agents adoucissants pharmaceutiquement acceptables étant présents, par gramme de sulfate de chondroïtine incorporé, en une quantité de 100 à 800 mg.

10. Formulation selon l'une des revendications 1 à 6, **caractérisée en ce que** l'agent de texture primaire est présent, par gramme de sulfate de chondroïtine incorporé, en une quantité de 1001 à 3000 mg, et l'agent de texture secondaire est présent, par gramme de sulfate de chondroïtine incorporé, en une quantité de 1500 à 2499 mg.

11. Formulation selon la revendication 10, dans laquelle les excipients supplémentaires sont constitués d'agents adoucissants pharmaceutiquement acceptables et/ou d'aromes et/ou d'agents de texture, lesdits excipients supplémentaires étant présents, par gramme de sulfate de chondroïtine incorporé, en une quantité de 30 à 800 mg.

12. Procédé pour obtenir les formulations selon une des revendications précédentes, comprenant le mélange de sulfate de chondroïtine avec de l'eau ou avec des solutions ou dispersions des excipients supplémentaires, comprenant des agents de texture, pour favoriser la pré-dissolution, de préférence à une température élevée, facultativement suivi par le séchage du mélange ainsi obtenu, jusqu'à ce que la teneur en eau soit celle prévue pour les formulations finales.
